# EUROPEAN PATENT APPLICATION

(11) **EP 3 492 572 A1**
(43) Date of publication of application: **05.06.2019**
(21) Application number: 17834202.8
(22) Date of filing: 21.07.2017
(51) Int. Cl.: C12M 1/00

(54) **GERM-FREE GLOVE BOX**

(30) Priority: 27.07.2016 JP 2016147250
(71) Applicant: Sinfonia Technology Co., Ltd., Tokyo 105-8564 (JP)
(72) Inventor: TAKEUCHI, Haruki, Tokyo 105-8564 (JP); YAMADA, Takayuki, Tokyo 105-8564 (JP); SAEKI, Toru, Tokyo 105-8564 (JP); ONISHI, Takumi, Tokyo 105-8564 (JP); UDA, Hiroyuki, Tokyo 105-8564 (JP); GOMI, Hisashi, Tokyo 107-6325 (JP); FUJI, Toshimitsu, Tokyo 107-6325 (JP); SHIRAIWA, Hirotsugu, Tokyo 107-6325 (JP); YAMAMOTO, Norihiko, Fuchu-shi Tokyo 183-8705 (JP)
(74) Representative: Cabinet Beau de Loménie
(86) International application number: PCT/JP2017/026545
(87) International publication number: WO 2018/021195

(57) **Abstract**

A germ-free glove box with which, firstly, outside spaces beside the box are effectively utilized, and secondly, a large working space is secured at around the bottom surface of a germ-free space is provided. A germ-free glove box 10 includes: a housing 12 in which a germ-free space 11 is formed, the housing 12 having a connector 19 to which a container 30 housing an object is connected; and a glove 17 which is provided at a front plate of the housing 12 to allow an operating personnel to perform an operation in the germ-free space 11 from the outside of the housing 12. In this germ-free glove box 10, the connector 19 is provided on an upper plate 12e of the housing 12.

## Description

### [Technical Field]

The present invention relates to a germ-free glove box in which gloves are provided to allow an operating personnel outside the box to perform operations in a germ-free space in the box.

### [Background Art]

As an example of such a germ-free glove box, a test isolator recited in Patent Literature 1 has been known (FIG. 1). The inside of a housing which constitutes the test isolator is a germ-free space, and gloves into which an operating personnel is able to insert arms is provided at the front plate of the housing. On a side plate of the housing, a connector is provided to connect the housing with a storage in which an object is stored. An operating personnel wearing the gloves opens a lid of the storage from the inside of the housing and takes the object out to the germ-free space. In this way, operations regarding the object can be performed in the germ-free space.

### [Citation List]

### [Patent Literatures]

[Patent Literature 1] Japanese Patent No. 4329066

### [Summary of Invention]

### [Technical Problem]

However, when the connector is provided on the side plate of the housing as in the case above, any other devices cannot be provided to be adjacent to the side face. Furthermore, a space for connecting the storage to the connector must be secured beside the housing. The footprint of the test isolator of Patent Literature 1 is therefore substantially large, and spaces beside the test isolator are not effectively utilized. In addition to the above, an operating personnel typically performs operations while placing the object and other tools on the bottom surface of the germ-free space. However, when the connector is provided on the side plate, nothing can be placed on the bottom surface in the vicinity of the connector because a space for, for example, opening and closing the lid of the storage must be secured. For this reason, a working space in the germ-free space is disadvantageously small.

In consideration of these problems, an object of the present invention is to provide a germ-free glove box with which, firstly, outside spaces beside the box are effectively utilized, and secondly, a large working space is secured at around the bottom surface of a germ-free space.

### [Solution to Problem]

According to the present invention, a housing in which a germ-free space is formed, the housing having a connector to which a container housing an object is connected; and a glove which is provided at a front plate of the housing to allow an operating personnel to perform an operation in the germ-free space from the outside of the housing are provided, and the connector is provided on an upper plate of the housing. According to the present invention, because the connector to which the container is connected is provided on the upper plate of the housing, no connector is provided on a side plate of the housing as in the conventional apparatuses, and hence outside spaces beside the housing are effectively utilized. Furthermore, because the connector is provided on the upper plate of the housing, operations such as opening and closing of the container are performed predominantly in an upper portion of the germ-free space. For this reason, a large working space can be secured in the vicinity of the bottom surface of the germ-free space.

The present invention may be arranged so that, a lifting mechanism configured to move up or down a bottom lid of the container together with the object; and operation units which are provided in the germ-free space to drive the lifting mechanism are further included, and the lifting mechanism becomes drivable when at least two of the operation units are simultaneously operated.

Because the operation units for driving the lifting mechanism are provided in the germ-free space, the operating personnel wearing the glove is able to operate the operation units without taking the glove off, and hence the operation can be effectively carried out. However, because the hands of the operating personnel are in the germ-free space while the lifting mechanism is in operation, there is a possibility that a hand of the operating personnel is pinched between the bottom lid and the connector. In this regard, because at least two of the operation units must be simultaneously operated to drive the lifting mechanism, the operating personnel operates the operation units by using both hands, and hence the operating personnel cannot move the hands at will while the lifting mechanism is in operation. This prevents a hand of the operating personnel from being pinched between the bottom lid and the connector as the operating personnel carelessly moves the hand while the lifting mechanism is in operation.

The present invention may be arranged so hat an introduction portion is formed in a side plate of the housing to introduce cleaned gas into the germ-free space, and the operation units are disposed not to overlap a locus of the object moving up or down, when viewed from a side.

The cleaned gas introduced through the introduction portion formed in the side plate of the housing flows in the germ-free space along a direction from this side plate to the other side plate (hereinafter, the airflow direction). In this regard, if, for example, the glove has a hole, germs adhered to the glove are conveyed by the flow of the cleaned gas during operation of the operation units, with the result that the object may be contaminated. When the operation units are disposed not to overlap the locus of the object moving up or down when viewed from the side as described above, no object is provided downstream in the airflow direction of the operation units, and hence adherence of germs flown from the vicinity of the operation units to the object is prevented. The present invention may be arranged so that the operation units are disposed on a ceiling surface of the germ-free space. When the operation units are provided on the ceiling surface of the germ-free space, a larger working space can be secured in the vicinity of the bottom surface of the germ-free space. Furthermore, when, for example, liquid is spilled in the germ-free space, the operation units do not get wet because the operation units are provided on the ceiling surface. The present invention is preferably arranged so that the operation units are provided on the front plate side as compared to the connector.

When the operation units are provided on the front plate side as compared to the connector, it is possible to prevent a hand or arm of the operating personnel operating the operation units from being interfered with the bottom lid and the object moving down from the connector.

### [Brief Description of Drawings]

FIG. 1 is a front view of a germ-free working system of an embodiment.
FIG. 2 is a cross section taken along a line II-II in FIG. 1.
FIG. 3 is a cross section taken along a line III-III in FIG. 2.
FIG. 4 is a cross section taken along a line IV-IV in FIG. 1.

### [Description of Embodiments]

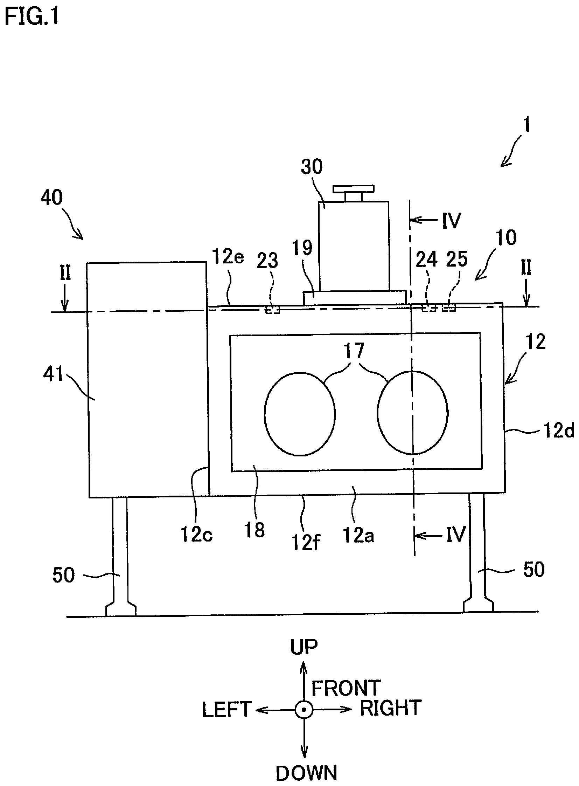

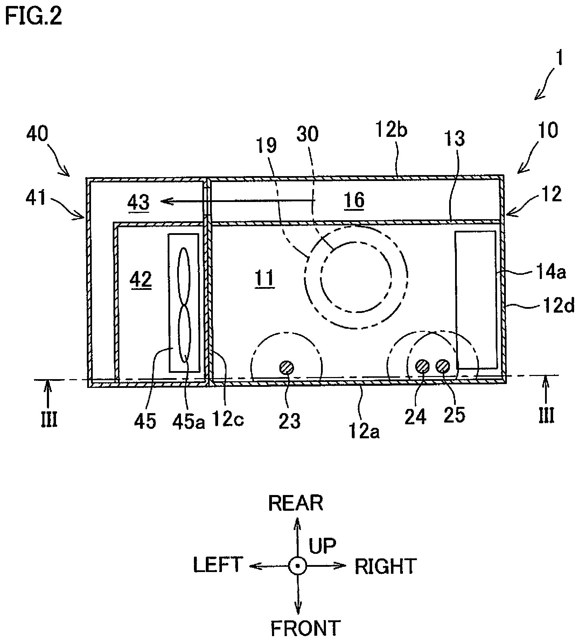

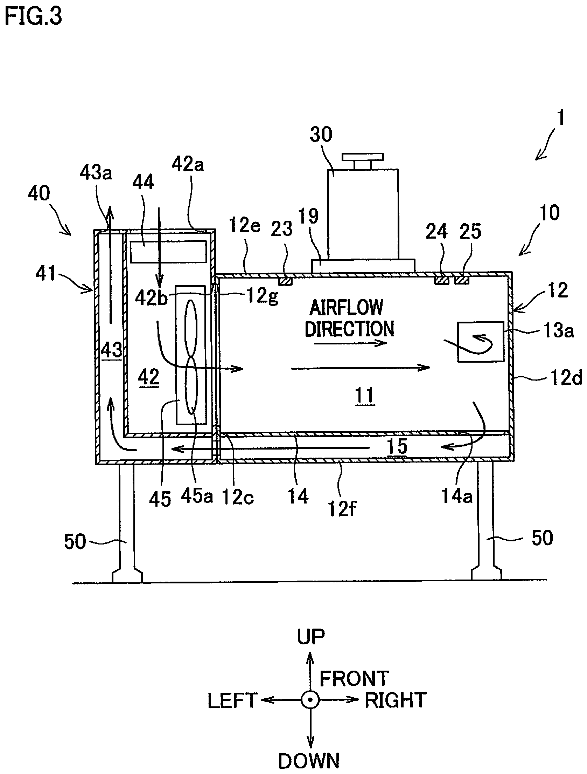

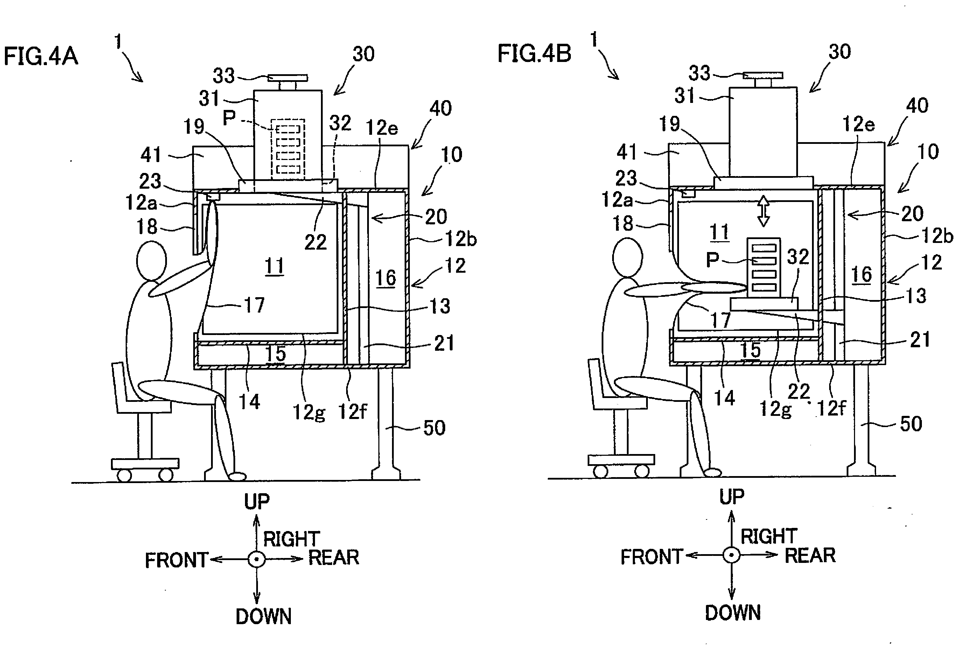

The following will describe an embodiment of a germ-free working system including a germ-free glove box of the present invention, with reference to figures. FIG. 1 is a front view of the germ-free working system of the present embodiment. FIG. 2 is a cross section taken along a line II-II in FIG. 1. FIG. 3 is a cross section taken along a line III-III in FIG. 2. FIG. 4 is a cross section taken along a line IV-IV in FIG. 1. Hereinafter, explanations will be given with reference to the directions shown in the figures.

### (Germ-Free Working System)

The germ-free working system 1 of the present embodiment is arranged such that a germ-free glove box 10 and an air purifier 40 are supported by a supporter 50. In the germ-free glove box 10, a germ-free space 11 is formed. This germ-free space 11 is maintained to be germ free by cleaned air (cleaned gas) supplied from the air purifier 40. While in the germ-free working system 1 of the present embodiment operations such as cell culture are performed in the germ-free space 11, operations performed in the germ-free space 11 are not limited to this. It is noted that the germ-free space in the present invention encompasses not only a space which is completely germ free but a space where a few germs not adversely affecting operations exist.

### (Germ-Free Glove Box)

The germ-free glove box 10 includes a rectangular parallelepiped housing 12 having a front plate 12a, a rear plate 12b, a left side plate 12c, a right side plate 12d, an upper plate 12e, and a lower plate 12f. As shown in FIG. 4, the inside of the housing 12 is divided into two in the front-rear direction by a partition wall 13 which is provided to extend in the vertical direction. The front space is further divided into two in the up-down direction by a partition floor 14 which extends in the horizontal direction. To put it differently, the internal space of the housing 12 is divided into the germ-free space 11 formed in front of the partition wall 13 and above the partition floor 14, a lower exhaust space 15 formed in front of the partition wall 13 and below the partition floor 14, and a rear exhaust space 16 formed behind the partition wall 13.

As shown in FIG. 3, in the left side plate 12c of the housing 12, an introduction portion 12g is formed to introduce cleaned air supplied from the air purifier 40 into the germ-free space 11. At a right end portion of the partition floor 14 a communication portion 14a is formed to allow the germ-free space 11 to communicate with the lower exhaust space 15, whereas at a right end portion of the partition wall 13 a communication portion 13a is formed to allow the germ-free space 11 to communicate with the rear exhaust space 16. With this arrangement, as indicated by the arrows in FIG. 3, cleaned air supplied from the air purifier 40 to the germ-free space 11 through the introduction portion 12g flows along an airflow direction which is from left to right in the germ-free space 11, and is then either discharged from the communication portion 14a to the lower exhaust space 15 or discharged from the communication portion 13a to the rear exhaust space 16. As shown in FIG. 1, two gloves 17 are provided in a region of the front plate 12a of the housing 12, which faces the germ-free space 11. The two gloves 17 are side by side in the left-right direction. Each glove 17 is, for example, a glove made of rubber, and does not allow air movement between the outside and the germ-free space 11 through the glove 17. A light-transmitting member 18 which is made of light-transmitting glass or resin is fitted to the most part of the front plate 12a including the region where the gloves 17 are provided. This light-transmitting member 18 makes it possible to observe the germ-free space 11 from the outside of the housing 12. With this arrangement, the operating personnel wearing the gloves 17 is able to perform an operation in the germ-free space 11 from the outside of the housing 12, while the germ-free space 11 is isolated from the outside air.

On the upper plate 12e of the housing 12, a connector 19 connected to a container 30 is provided in a region facing the germ-free space 11. The container 30 will be described first. As shown in FIG. 4, the container 30 is arranged to be able to store plural cell containers P each storing a cell. The container 30 has a cylindrical main body 31 which is open at a lower end portion and a disc-shaped bottom lid 32 which is provided for closing the opening at the lower end portion of the main body 31. The cell containers P are placed on the bottom lid 32, and are moved up or down together with the bottom lid 32, by a later-described lifting mechanism.

The connector 19 is provided with an unillustrated opening/closing mechanism for opening and closing the bottom lid 32. This opening/closing mechanism is able to switch the bottom lid 32 between a closed state in which the main body 31 is closed in an airtight manner and an open state in which the lid is separated from the main body 31, by rotating the bottom lid 32 relative to the main body 31. Furthermore, a lifting mechanism 20 is provided in the housing 12 to move up or down the bottom lid 32. The lifting mechanism 20 is a ball screw mechanism and includes a screw shaft 21 extending in the vertical direction and a platform 22 which is movable in the up-down direction along the screw shaft 21. The screw shaft 21 is provided in the rear exhaust space 16. A part of the platform 22 attached to the screw shaft 21 reaches the germ-free space 11 through an unillustrated slit (gap) formed in the partition wall 13. With this arrangement, the bottom lid 32 detached from the main body 31 by the opening/closing mechanism is able to move up or down while being supported by the platform 22. Dust or the like may be generated from around the screw shaft 21 when the lifting mechanism 20 is driven. However, because an airflow is generated toward the rear exhaust space 16 from the germ-free space 11 through the slit, entrance of dust or the like into the germ-free space 11 is prevented. The bottom lid 32 of the container 30 is made of a magnetic material, and the connector 19 is provided with an unillustrated electromagnet. Because the bottom lid 32 is attracted to the connector 19 on account of the electromagnet, positioning of the container 30 and connection to the connector 19 can be easily done. Furthermore, because the magnetic force of the electromagnet is evenly exerted, the close contactness (sealing performance) between components is improved and an outside air shielding effect is improved.

The container 30 may be conveyed by the operating personnel and connected to the connector 19, or may be automatically conveyed to the connector 19 by an unillustrated conveyor. An example of the conveyor is an OHT (Overhead Hoist Transfer) which runs along a track mounted on the ceiling of the facility and includes an elevatable hoist mechanism. When the container 30 is conveyed by the OHT, a flange 33 (see FIG. 4) which can be gripped by the hoist mechanism of the OHT is preferably provided at an upper portion of the container 30. In the germ-free glove box 10 of the present embodiment, the connector 19 to which the container 30 is connected is provided on the upper plate 12e of the housing 12. Handover of the container 30 between the OHT and the connector 19 is therefore easily done.

### (Operation of Lifting Mechanism)

In the germ-free glove box 10 of the present embodiment, three operation units 23 to 25 are provided in the germ-free space 11 to drive the lifting mechanism 20. The operation units 23 to 25 are embodied as, for example, switch buttons, and are provided at locations where the operating personnel wearing the gloves 17 is able to operate them. The operation units 23 to 25 are not limited to buttons, and may be lever switches or sensor switches, for example.

Specific locations of the operation units 23 to 25 will be described. The operation units 23 to 25 are provided on a ceiling surface of the germ-free space 11 (i.e., the inner surface of the upper plate 12e of the housing 12) and are lined up in the left-right direction to form a single line. As shown in FIG. 2, the operation units 23 to 25 are provided in the vicinity of the front plate 12a of the housing 12, i.e., are provided on the front plate 12a side as compared to the connector 19. As a result, as clearly shown in FIG. 4, the operation units 23 to 25 do not overlap the locus of the bottom lid 32 and the cell containers P moving up or down (i.e., a region directly below the connector 19), when viewed from the side.

As shown in FIG. 2, the operation unit 23 is provided to the left of the connector 19, whereas the operation units 24 and 25 are provided to the right of the connector 19 and neighbor each other. On the contrary, the operation unit 23 may be provided to the right of the connector 19 whereas the operation units 24 and 25 may be provided to the left of the connector 19. The operation unit 23 is a button by which the lifting mechanism 20 is made drivable, the operation unit 24 is a button by which the platform 22 is moved up, and the operation unit 25 is a button by which the platform 22 is moved down. The functions of the operation units 23 to 25 are suitably exchangeable. For example, the functions of the operation units 24 and 25 may be swapped. Because the operation unit 23 is sufficiently separated from the operation units 24 and 25, it is impossible to press the operation unit 24 or 25 while pressing the operation unit 23, by using one hand. For this reason, the operating personnel cannot move up or down the platform 22 unless he/she keeps operating (pressing) the operation unit 23 by the left hand while keeping operating (pressing) the operation unit 24 or the operation unit 25 by the right hand. One-dot chain lines in FIG. 2 indicate arcs centered on the respective operation units 23 to 25, and each of the arcs roughly indicates the reach range of one hand or arm of a typical adult male who is operating each of the operation units 23 to 25 by one hand. As clearly shown in FIG. 2, the hands or arms operating the operation units 23 to 25 are positioned not to reach the connector 19. It is therefore possible to avoid the risk that a hand (finger in particular) or an arm is pinched between the bottom lid 32 and the connector 19 when the operating personnel operating the operation units 23 to 25 closes the bottom lid 32.

### (Air Purifier)

The air purifier 40 is configured to supply cleaned air to the germ-free space 11 in the germ-free glove box 10 and is provided adjacent to the left side plate 12c of the housing 12 of the germ-free glove box 10. The air purifier 40 includes a rectangular parallelepiped casing 41. The internal space of the casing 41 is divided into a cleaning space 42 and an exhaust space 43.

As shown in FIG. 3, an intake portion 42a is formed in the upper face of the cleaning space 42. Outside air can be introduced into the cleaning space 42 through the intake portion 42a. In a right side face of the cleaning space 42 (i.e., the face in contact with the germ-free glove box 10), an air supply portion 42b is formed. The air supply portion 42b communicates with the introduction portion 12g of the germ-free glove box 10. Cleaned air is supplied from the air purifier 40 to the germ-free space 11 via the air supply portion 42b and the introduction portion 12g.

In the cleaning space 42, a filter 44 for purifying air and an FFU (Fan Filter Unit) 45 are provided. The FFU 45 is configured to further purify the air having passed the filter 44 and supply the cleaned air to the germ-free space 11. The filter 44 is provided directly below the intake portion 42a. Air taken through the intake portion 42a is cleaned first by the filter 44. The FFU 45 is provided below the filter 44 to be adjacent to the air supply portion 42b. As a fan 45a of the FFU 45 rotates, cleaned air flows in the airflow direction (from left to right) . Therefore, as indicated by the arrows in FIG. 3, air taken into the cleaning space 42 through the intake portion 42a is purified by the filter 44 and the FFU 45 and is then fed in the airflow direction by the fan 45a of the FFU 45 to the germ-free space 11 via the air supply portion 42b and the introduction portion 12g.

An exhaust portion 43a is formed in the upper face of the exhaust space 43. Air can be exhausted from the exhaust space 43 to the outside through the exhaust portion 43a. The exhaust space 43 communicates with the lower exhaust space 15 and the rear exhaust space 16 of the germ-free glove box 10. As indicated by the arrows in FIG. 2 and FIG. 3, air flows from the lower exhaust space 15 and the rear exhaust space 16 to the exhaust space 43.

In the germ-free working system 1 arranged as described above, a circulation path of air is formed so that air flows as follows: outside → the intake portion 42a → the filter 44 → the FFU 45 → the air supply portion 42b → the introduction portion 12g → the germ-free space 11 → communication portion 14a (or the communication portion 13a) → the lower exhaust space 15 (or the rear exhaust space 16) → the exhaust space 43 → the exhaust portion 43a → outside. In each figure, each of the introduction portion 12g, the communication portion 13a, the communication portion 14a, the intake portion 42a, the air supply portion 42b, and the exhaust portion 43a is depicted as a large single opening for simplification. In reality, each of these portions is formed of a member such as a porous plate having holes or a mesh.

### (Flow of Operation)

The following will describe a flow of an operation performed by using the germ-free working system 1 arranged as described above. To begin with, before the start of the operation, the germ-free glove box 10 and the air purifier 40 are decontaminated by using decontamination gas, etc., according to need. Subsequently, the air purifier 40 is activated to cause the germ-free space 11 of the germ-free glove box 10 to be germ free. The pressure in the germ-free space 11 is maintained to be slightly higher than the atmospheric pressure (i.e., to be positive pressure) in order to prevent the outside air from entering the germ-free space 11. The container 30 storing cell containers P which are operation targets is decontaminated in advance.

Subsequently, the container 30 having been decontaminated is connected to the connector 19. As described above, this operation may be performed by the operating personnel or may be performed by a conveyor when the conveyor is provided in the facility. Once the container 30 is connected to the connector 19, the bottom lid 32 of the container 30 is detached from the main body 31 by the opening/closing mechanism. Subsequently, as shown in FIG. 4(a), the operating personnel wearing the gloves 17 operates the operation unit 23 by the left hand and operates the operation unit 25 by the right hand. As a result, the platform 22 is moved down together with the bottom lid 32 and the cell containers P and is eventually placed in the germ-free space 11.

As shown in FIG. 4(b), when the platform 22 is moved down to a predetermined position, the operating personnel detaches the hands from the operation units 23 and 25 and performs a predetermined operation for the cell containers P in the germ-free space 11. After the end of the operation, the operating personnel operates the operation unit 23 by the left hand and operates the operation unit 24 by the right hand, so as to move up the platform 22 together with the bottom lid 32 and the cell containers P. Once the bottom lid 32 is moved up to a position where the main body 31 of the container 30 is closable, the bottom lid 32 is closed by the opening/closing mechanism. Lastly, the container 30 storing the cell containers P for which the predetermined operation has been done is conveyed to the next processing apparatus or the like by the operating personnel or the conveyor. This is the end of the flow of the operation. After the end of the operation, the container 30 may be decontaminated.

### (Effects)

In the germ-free glove box 10 of the present embodiment, because the connector 19 to which the container 30 is connected is provided on the upper plate 12e of the housing 12, no connector is provided on a side plate as in the conventional apparatuses, and hence outside spaces beside the housing 12 are effectively utilized. Furthermore, because the connector 19 is provided on the upper plate 12e of the housing 12, operations such as opening and closing of the container 30 are performed predominantly in an upper portion of the germ-free space 11. For this reason, a large working space can be secured in the vicinity of the bottom surface of the germ-free space 11 (i.e., the upper surface of the partition floor 14) . Furthermore, when the container 30 is connected to a side plate of the housing 12, the container 30 is cantilevered and hence it is difficult to connect a heavy container 30. In this regard, because in the present embodiment the container 30 is placed on the upper plate 12e, the container 30 is stably supported even when the container 30 is heavy.

In the present embodiment, the lifting mechanism 20 configured to move up or down the bottom lid 32 of the container 30 together with the cell containers P (objects to be stored) and the operation units 23 to 25 provided in the germ-free space 11 to drive the lifting mechanism 20 are further provided. The lifting mechanism 20 becomes drivable when at least two of the operation units 23 to 25 are simultaneously operated. Because the operation units 23 to 25 for driving the lifting mechanism 20 are provided in the germ-free space 11, the operating personnel wearing the gloves 17 is able to operate the operation units 23 to 25 without taking the gloves 17 off, and hence the operation can be effectively carried out. However, because the hands of the operating personnel are in the germ-free space 11 while the lifting mechanism 20 is in operation, there is a possibility that a hand of the operating personnel is pinched between the bottom lid 32 and the connector 19. In this regard, because at least two of the operation units 23 to 25 must be simultaneously operated to drive the lifting mechanism 20, the operating personnel operates the operation units 23 to 25 by using both hands, and hence the operating personnel cannot move the hands at will while the lifting mechanism 20 is in operation. This prevents a hand of the operating personnel from being pinched between the bottom lid 32 and the connector 19 as the operating personnel carelessly moves the hand while the lifting mechanism 20 is in operation.

In the present embodiment, the introduction portion 12g through which cleaned air (cleaned gas) is introduced into the germ-free space 11 is formed in the side plate 12c of the housing 12, and the operation units 23 to 25 are disposed not to overlap the locus of the cell containers P moving up or down, when viewed from the side. The cleaned air introduced through the introduction portion 12g formed in the side plate 12c of the housing 12 flows in the germ-free space 11 along the airflow direction which is from the side plate 12c to the other side plate 12d. In this regard, if, for example, a glove 17 has a hole, germs adhered to the glove 17 are conveyed by the flow of the cleaned air during operation of the operation units 23 to 25, with the result that the cell containers P may be contaminated. When the operation units 23 to 25 are disposed not to overlap the locus of the cell containers P moving up or down when viewed from the side as described above, no cell container P is provided downstream in the airflow direction of the operation units 23 to 25, and hence adherence of germs flown from the vicinity of the operation units 23 to 25 to the cell containers P is prevented.

Furthermore, in the present embodiment, because the operation units 23 to 25 are provided on the ceiling surface of the germ-free space 11 (i.e., the inner surface of the upper plate 12e of the housing 12), a large working space can be secured in the vicinity of the bottom surface of the germ-free space 11 (i.e., the upper surface of the partition floor 14). Furthermore, when, for example, liquid is spilled in the germ-free space 11, the operation units 23 to 25 do not get wet because the operation units 23 to 25 are provided on the ceiling surface. Furthermore, it is possible to prevent generation of germs or adhesion of dust due to intrusion of spilled liquid into gaps around the operation units 23 to 25. In the present embodiment, because the operation units 23 to 25 are provided on the front plate 12a side as compared to the connector 19, it is possible to prevent a hand or arm of the operating personnel operating the operation units 23 to 25 from being interfered with the bottom lid 32 and the cell containers P moving down from the connector 19.

### (Other Embodiments)

A preferred embodiment of the present invention has been described. It should be noted that the present invention is not limited to the above-described embodiment, and various changes, substitutions, and alterations can be made herein without departing from the spirit and scope of the invention as defined by the appended claims, as described below, for example.

For example, while in the embodiment above the operation units 23 to 25 for driving the lifting mechanism 20 are provided in the germ-free space 11, the operation units 23 to 25 may be provided outside the housing 12. In this case, while the operating personnel is required to take off the gloves 17 each time he/she operates the operation units 23 to 25, accidents such as pinching of a hand of the operating personnel do not occur. The operation units 23 to 25 may be disposed at an inner surface other than the ceiling surface of the germ-free space 11. For example, the operation units 23 to 25 may be provided on the inner surface of the front plate 12a of the housing 12 or the light-transmitting member 18.

While in the embodiment above the lifting mechanism 20 is a ball screw mechanism, the lifting mechanism 20 may employ a mechanism of another type.

In the embodiment above, the bottom lid 32 of the container 30 is opened or closed as the bottom lid 32 is rotated by the opening/closing mechanism provided at the connector 19. In this connection, the specific structure of the bottom lid 32 is not limited to the rotation type, and accordingly, the specific structure of the opening/closing mechanism is suitably changeable.

While in the embodiment above the air purifier 40 is provided adjacent to the side plate 12c, the air purifier 40 may not be disposed in this way. The air purifier 40 may be omitted when there is another means which is able to maintain the germ-free space 11 to be germ free.

In the embodiment above, the air purifier 40 supplies cleaned air as cleaned gas. The cleaned gas is not limited to air, and may be gas such as nitrogen gas or carbon dioxide gas, or may be a mixture of plural types of gases. When gas other than air is used, it is necessary to connect the intake portion 42a with the exhaust portion 43a by a duct or the like.

In the embodiment above, one germ-free space 11 is formed in one germ-free glove box 10. Alternatively, plural germ-free spaces 11 may be formed in one germ-free glove box 10. For example, two germ-free spaces 11 are provided in one germ-free glove box 10 to communicate with each other, and the connector 19 and the lifting mechanism 20 are provided in each germ-free space 11. This arrangement facilitates operations such as transfer of cell containers P from a container 30 connected to one germ-free space 11 to a container 30 connected to the other germ-free space 11.

The germ-free glove box 10 may be connected to another device such as an incubator for culturing cells and a conveyor for conveying cell containers P. In such cases, a door which is openable and closable is preferably provided between the germ-free space 11 of the germ-free glove box 10 and another device.

### [Reference Signs List]

- 1: germ-free working system
- 10: germ-free glove box
- 11: germ-free space
- 12: housing
- 12g: introduction portion
- 17: glove
- 19: connector
- 20: lifting mechanism
- 23: to 25 operation unit
- 30: container
- 32: bottom lid
- P: cell container (object)

## Claims

1. A germ-free glove box comprising:
a housing in which a germ-free space is formed, the housing having a connector to which a container housing an object is connected; and
a glove which is provided at a front plate of the housing to allow an operating personnel to perform an operation in the germ-free space from the outside of the housing,
the connector being provided on an upper plate of the housing.

2. The germ-free glove box according to claim 1, further comprising:
a lifting mechanism configured to move up or down a bottom lid of the container together with the object; and
operation units which are provided in the germ-free space to drive the lifting mechanism,
the lifting mechanism becoming drivable when at least two of the operation units are simultaneously operated.

3. The germ-free glove box according to claim 2, wherein,
an introduction portion is formed in a side plate of the housing to introduce cleaned gas into the germ-free space, and
the operation units are disposed not to overlap a locus of the object moving up or down, when viewed from a side.

4. The germ-free glove box according to claim 2 or 3, wherein, the operation units are disposed on a ceiling surface of the germ-free space.

5. The germ-free glove box according to any one of claims 2 to 4, wherein, the operation units are provided on the front plate side as compared to the connector.
